# EUROPÄISCHE PATENTSCHRIFT

(11) **EP 1 430 301 B1**
(45) Veröffentlichungstag und Bekanntmachung des Hinweises auf die Patenterteilung: **09.01.2008**
(21) Anmeldenummer: 02799414.4
(22) Anmeldetag: 19.09.2002
(51) Int. Cl.: G01N 33/24

(54) **VERFAHREN ZUR HERSTELLUNG EINER NORMALISIERTEN GENBANK AUS NUKLEINSÄURE-EXKTRAKTEN VON BODENPROBEN UND DEREN VERWENDUNG**
METHOD FOR PRODUCING A NORMALIZED GENE LIBRARY FROM NUCLEIC ACID EXTRACTS OF SOIL SAMPLES AND THE USE THEREOF
PROCEDE DE PRODUCTION D'UNE BANQUE DE GENES NORMALISEE A PARTIR D'EXTRAITS D'ACIDES NUCLEIQUES PROVENANT D'ECHANTILLONS DE SOL ET SON UTILISATION

(30) Priorität: 21.09.2001 DE 10146572
(43) Veröffentlichungstag der Anmeldung: 23.06.2004
(73) Patentinhaber: BASF Aktiengesellschaft, 67056 Ludwigshafen (DE)
(72) Erfinder: HAUER, Bernhard, 67136 Fussgöheim (DE); MATUSCHEK, Markus, 69469 Weinheim (DE); SCHMID, Rolf, 70329 Stuttgart (DE); BUTA, Christiane, 70195 Stuttgart (DE); KAUFFMANN, Isabelle, 70327 Stuttgart (DE); LÄMMLE, Katrin, 71665 Vaihingen (DE); ZIPPER, Hubert, 70597 Stuttgart (DE)
(74) Vertreter: Fitzner, Uwe
(86) Internationale Anmeldenummer: PCT/EP2002/010510
(87) Internationale Veröffentlichungsnummer: WO 2003/027669

(56) Entgegenhaltungen:
- WO-A-98/58085
- MATTHEY B ET AL: "A new series of pET-derived vectors for high efficiency expression of Pseudomonas exotoxin-based fusion proteins" GENE, ELSEVIER BIOMEDICAL PRESS. AMSTERDAM, NL, Bd. 229, Nr. 1-2, 18. März 1999 (1999-03-18), Seiten 145-153, XP004161169 ISSN: 0378-1119
- HOHEISEL D: "A CASSETTE WITH SEVEN UNIQUE RESTRICTION SITES, INCLUDING OCTANUCLEOTIDE SEQUENCES: EXTENSION OF MULTIPLE-CLONING-SITE PLASMIDS" GENE, ELSEVIER BIOMEDICAL PRESS. AMSTERDAM, NL, Bd. 80, Nr. 1, 1989, Seiten 151-154, XP001154198 ISSN: 0378-1119
- HELFRICH W ET AL: "A rapid and versatile method for harnessing scFv antibody fragments with various biological effector functions" JOURNAL OF IMMUNOLOGICAL METHODS, ELSEVIER SCIENCE PUBLISHERS B.V.,AMSTERDAM, NL, Bd. 237, Nr. 1-2, April 2000 (2000-04), Seiten 131-145, XP004192501 ISSN: 0022-1759
- PATANJALI S R ET AL: "CONSTRUCTION OF A UNIFORM-ABUNDANCE (NORMALIZED) CDNA LIBRARY" PROCEEDINGS OF THE NATIONAL ACADEMY OF SCIENCES OF USA, NATIONAL ACADEMY OF SCIENCE. WASHINGTON, US, Bd. 88, Nr. 5, 1. März 1991 (1991-03-01), Seiten 1943-1947, XP000368687 ISSN: 0027-8424
- SOARES M B ET AL: "Construction and characterization of a normalized cDNA library" PROCEEDINGS OF THE NATIONAL ACADEMY OF SCIENCES OF USA, NATIONAL ACADEMY OF SCIENCE. WASHINGTON, US, Bd. 91, September 1994 (1994-09), Seiten 9228-9232, XP002138140 ISSN: 0027-8424

## Beschreibung

Gegenstand der Erfindung ist ein Verfahren zur Herstellung einer normalisierten Genbank aus Nukleinsäure-Extrakten von Bodenproben sowie deren Verwendung.

Enzyme aus Mikroorganismen besitzen ein breites Anwendungspotential. Im medizinisch-pharmazeutischen Bereich werden Enzyme z. B. bei der Wirkstoffsuchforschung und der Entwicklung von molekularbiologischen Testsystemen eingesetzt. Enzyme werden bei der Synthese von Antibiotika und Antibiotikaderivaten, zur Herstellung von Hormonen sowie als Zusatzstoffe in der Lebensmittelindustrie, in der Waschmittelindustrie und als Katalysatoren zur Produktion von Chemikalien verwendet, um nur einige Beispiele zu nennen. Um die derzeitigen enzymatischen Verfahren zu verbessern und neue Anwendungsfelder für Enzyme zu erschließen, müssen vorhandene Enzyme optimiert und neue Enzyme im Screening selektiert werden.
Bisher wurde das Screening nach neuen Enzymen dadurch begrenzt, daß ausschließlich Reinkulturen von Mikroorganismen durchmustert wurden. Es konnte jedoch gezeigt werden, daß lediglich ca. 1 % aller Mikroorganismen kultivierbar sind, 99 % der Mikroorganismen sind mit den derzeit bekannten Methoden nicht als reine Stämme kultivierbar. Folglich standen letztere Organismen bisher nicht für die Isolierung von neuen Enzymen zur Verfügung. In Genbanken aus Nukleinsäuren von verschiedenen Umweltstandorten werden theoretisch alle darin vorkommenden Enzyme erfaßt, ohne daß die betreffenden Spenderorganismen isoliert werden müssen.
Ein Verfahren zur Herstellung von Genbanken aus Umweltproben muß bestimmte Anforderungen erfüllen:
- Mit dem Verfahren muß DNA aus allen in der Probe vertretenen Spezies isoliert werden können.
- Die DNA muß zur Erstellung der Genbank nach der isolierung intakt sein und darf nicht durch die verschiedenen Reinigungs- und Isolierungs-Prozesse beschädigt werden.
- Das Verfahren muß unabhängig von der Zusammensetzung der Bodenprobe sowie der Population von Mikroorganismen sein.

Kritisch ist dabei, z.B. ein Gleichgewicht zwischen einem umfassenden Zellaufschluß einerseits und möglichst geringer Zerstörung der DNA durch Scherkräfte andererseits herzustellen. Beispiele für die Isolierung von DNA aus Bodenproben sind bei Moré et al. (Appl. Environm. Microbiol., May 1994, 1572-1580) und Zhou et al. (Appl. Environm. Microbiol., Feb. 1996, 316-322) beschrieben. Allerdings werden hier die Nukleinsäuren entweder in ihrer Gesamtheit, d.h. nicht-selektiv, aus dem organischen Material extrahiert oder es wird lediglich DNA aus Gram-positiven Organismen isoliert.

Die isolierte DNA muß klonierbar sein. Ein Problem bei der DNA-Isolierung aus Boden ist beispielsweise, daß die Nukleinsäurepräparationen in verstärktem Maße Huminstoffe enthalten, die die weitere Aufbereitung der Nukleinsäuren, z. B. die Quantifizierung oder die enzymatische Weiterbehandlung, stark behindern oder sogar unmöglich machen.

Darüber hinaus gilt es sicherzustellen, daß in der isolierten Nukleinsäure-Population diejenigen Nukleinsäure-Spezies, die von Natur aus seltener vertreten sind, bei der weiteren Aufarbeitung, wie z. B. der Klonierung in geeignete Vektoren zur Erstellung einer Genbank, nicht verloren gehen. Dies kann durch die Herstellung einer normalisierten Genbank erreicht werden, bei deren Erstellung häufig vorkommende DNA-Spezies abgereichert und selten vorkommende DNA-Spezies angereichert werden.

Methoden zur Anreicherung selten vorkommender DNA-Spezies sind zahlreich aus der Literatur bekannt: Beispielhaft sind WO 95/08647, WO 95/11986, WO 97/48717 und WO 99/45154 genannt.

Die WO 95/08647 offenbart zunächst die Herstellung einer cDNA-Genbank in einem geeigneten Vektor und die Bereitstellung der Plasmide in ihrer einzelsträngigen Form durch Denaturierung. Anschließend erfolgt die Herstellung von Fragmenten, die komplementär zu nicht-kodierenden 3'-Regionen der einzelsträngigen Plasmide sind sowie deren Hybridisierung mit der cDNA-Genbank. Die Selektion beruht dabei auf dem Prinzip, daß die nicht-kodierenden 3'-Bereiche gegenüber kodierenden DNA-Bereichen, die häufig konserviert sind, statistisch gesehen seltener im Genom vertreten sind. Die gebildeten Hybride werden aufgereinigt und weiteren Denaturierungs- und Reassoziationszyklen unterworfen. Das zuvor beschriebene Vorgehen verlangt jedoch detaillierte Kenntnisse hinsichtlich der nicht-kodierenden Nukleotidsequenzen. Die WO 95/08647 verfolgt das Ziel, einen normalisierten menschlichen cDNA-Katalog bereitzustellen.. Dabei wird von Säugerzellen, insbesondere von Zellen aus Gehirn, Lunge oder Herz ausgegangen. Die Isolierung von mikrobieller genomischer DNA aus Bodenproben wird nicht erwähnt, vielmehr ist das Ausgangsmaterial der WO 95/08647 isolierte mRNA.

Die WO 95/11986 offenbart ein Verfahren zur Herstellung einer subtraktiven cDNA-Genbank, bei der ebenfalls die in Form von cDNA vorliegende Gesamt-DNA in einem ersten Schritt in einen Vektor kloniert wird. Nachfolgend wird diese DNA denaturiert und die einzelsträngige cDNA zur Hybridisierung mit dem spezifisch markierten Nukleinsäuremolekül eingesetzt, das aus der Gesamtheit der DNA subtrahiert werden soll. Durch die Abtrennung der gebildeten markierten DNA-Hybride entsteht eine subtraktive DNA-Bank. Eine Anreicherung seltener vorkommender Nukleinsäure-Spezies in der verbleibenden DNA-Bank wird dadurch jedoch nicht erreicht. Ferner wird die hier eingesetzte DNA aus Säugerzellen, insbesondere Tumorzellen, isoliert, wobei das verwendete Ausgangsmaterial isolierte mRNA ist. Die Isolierung mikrobieller genomischer DNA und deren Normalisierung wird nicht erwähnt.

Gegenüber den bislang erläuterten Methoden zur Herstellung subtraktiver Genbanken mittels Hybridisierung mit dazu hergestellten Sonden oder Nukleinsäurefragmenten wird in der WO 97/48717 die Herstellung einer normalisierten DNA-Genbank offenbart, wobei als Ausgangsmaterial genomische DNA aus nicht-kultivierbaren Organismen, z.B. aus Bodenproben, verwendet wird. Hier wird die DNA durch Proteinase K und "freeze-thaw"-Verfahren isoliert, anschließend über einen CsCl-Gradienten gereinigt und über PCR angereichert, die Komplexität der Genbank durch 16S-rRNA-Analyse untersucht und schließlich durch Denaturierung und Reassoziation für 12-36 Stunden bei 68 °C normalisiert. Diese US-Schrift weist jedoch den Mangel auf, daß keine Angaben darüber gemacht werden, wann der optimale Zeitpunkt zur Beendigung der Reassoziation tatsächlich eintritt, was jedoch entscheidend für die optimale Ausbeute an seltener vertretenen DNA-Spezies ist. Letzteres gilt ebenfalls für das Dokument WO 99/45154.

Weiterhin problematisch bei der Bereitstellung einer normalisierten Genbank ist, daß die Verfügbarkeit geeigneter Erkennungsstellen für Restriktionsendonukleasen zwecks Klonierung der DNA-Fragmente in einen geeigneten Vektor stark limitiert ist. Dies liegt vor allem darin begründet, daß die einen bestimmten Größenbereich umfassenden, isolierten DNA-Fragmente im Zuge der Vorbereitung auf die Klonierung nicht nochmals fragmentiert werden sollen. Aufgrund dessen werden die isolierten DNA-Fragmente in herkömmlichen Verfahren einer enzymatischen Methylierung unterzogen, wodurch die DNA vor dem Angriff von Restriktionsendonukleasen geschützt werden soll. Problematisch ist jedoch, daß diese Methylierung sehr aufwendig ist und zudem nicht mit 100%iger Sicherheit gleichmäßig über die gesamte DNA verteilt erfolgt, so daß der Schutz vor dem Angriff von Restriktionsendonukleasen in der Praxis nur unzufriedenstellend ist (Robbins, P.W. et al. (1992) Gene 111: 69-76).

Aufgabe der vorliegenden Erfindung ist es, ein Verfahren zur Herstellung von Genbanken zur Verfügung zu stellen und Genkonstrukte sowohl aus nicht-kultivierbaren als auch aus kultivierbaren Organismen zu liefern. Das erfindungsgemäße Verfahren soll insbesondere die Möglichkeit zur Herstellung von Genbanken aus Bodenproben bieten, um auch selten vorkommende DNA aus bisher im Labor nicht kultivierbaren Organismen bereitzustellen. Eine weitere Aufgabe der vorliegenden Erfindung ist die Identifizierung neuer Biokatalysatoren aus Bodenproben.

Diese Aufgabe wird erfindungsgemäß gelöst durch ein Verfahren zur Herstellung einer normalisierten Genbank aus Nukleinsäure-Extrakten von Bodenproben, wobei
a) Nukleinsäuren aus in Bodenproben enthaltenen Lebewesen extrahiert werden,
b) eine Fragmentierung der Nukleinsäuren erfolgt,
c) eine Quantifizierung der Nukleinsäurefragmente mittels Fluoreszenzfarbstoffen erfolgt,
d) eine Normalisierung der Nukleinsäurefragmente erfolgt, wobei die Nukleinsäurefragmente zunächst denaturiert werden und der Verlauf der Renaturierung mittels Fluoreszenzfarbstoffen verfolgt wird,
e) nach Beendigung der Renaturierung eine Trennung der doppelsträngig vorliegenden Nukleinsäuren von den einzelsträngig vorliegenden Nukleinsäuren durch Adsorptionschromatographie erfolgt, wobei die in der Fraktion der einzelsträngigen Nukleinsäuren enthaltenen Nukleinsäure-Spezies annähernd gleich häufig (normalisiert) vorliegen,
f) zur Erstellung der Genbank die normalisierten Nukleinsäure-Spezies in einen Vektor kloniert werden.

Ein Vorteil der vorliegenden Erfindung ist, daß die Nukleinsäuren aus den Bodenproben extrahiert, fragmentiert, quantifiziert, normalisiert und dann in einen zur Klonierung, Amplifikation und/oder Expression geeigneten Vektor kloniert werden. Wie nachfolgend noch ausgeführt wird, ist erfindungsgemäß eine Methylierung der isolierten DNA zum Schutz vor ungewünschter Fragmentierung durch Restriktionsendonukleasen nicht erforderlich, da die Fragmentierung vor der Normalisierung erfolgt. Erfindungsgemäß vorteilhaft werden nach der Fragmentierung spezielle "Linker" an die Restriktionsfragmente angefügt, die Erkennungssequenzen für extrem selten schneidende Restriktionsendonukleasen besitzen. Dadurch wird das erfindungsgemäße Verfahren bei gleichzeitiger Effizienzsteigerung der Genbankherstellung erheblich vereinfacht.

Ein weiterer Vorteil des erfindungsgemäßen Verfahrens ist dabei, daß Nukleinsäuren aus im Boden lebenden Organismen extrahiert werden, die bisher im Labor nicht kultiviert wurden. Als Beispiele für bisher nicht kultivierbare, bekannte Mikroorganismen sind zu nennen: Bakterien im Pansen der Wiederkäuer, obligate Endosymbionten von Protozoen und Insekten, das magnetotaktische Bakterium Achromatium oxaliferum (Amann, R.I. et al. (1995) Microbiol. Rev. 59: 143-169).

Das erfindungsgemäße Verfahren zeichnet sich dabei dadurch aus, daß eine selektive Isolierung von Nukleinsäuren aus Actinomyceten erfolgt. Die genaue Kenntnis oder zumindest der gezielte Ausschluß von Organismengruppen aus Bodenproben ist insofern von Vorteil, als daß die Auswahl eines geeigneten Wirtsorganismus, in den die isolierten DNA-Fragmente ggf. später übertragen werden sollen (z.B. zwecks Klonierung oder Funktionalitätskontrolle durch Expression), optimal vorgenommen werden kann.

In einer vorteilhaften Variante der vorliegenden Erfindung erfolgt zunächst eine DNA-Isolierung nach einem erfindungsgemäß modifizierten Protokoll von Zhou et al. (1996, Appl. Environ. Microbiol., 62 (2): 316-322), wobei die Modifikationen darin bestehen, daß die Freeze-Thaw-Zyklen vor der Proteinase-K-Behandlung durchgeführt werden. Diese Art der Probenaufbereitung ermöglicht es, eine Isolierung von DNA aus Actinomyceten nahezu auszuschließen. D.h., die auf diese Weise isolierte DNA ist vorteilhaft zur Übertragung in Gram-negative Mikroorganismen, wie z.B. E. coli, geeignet.
Ein besonderer Vorteil des erfindungsgemäßen Verfahrens zur Herstellung einer normalisierten Genbank aus Bodenproben ist, daß die DNA-Isolierung derart gesteuert werden kann, daß die DNA-Isolierung hinsichtlich der in Bodenproben vorkommenden Organismengruppen selektiv erfolgt. So ist es erfindungsgemäß z.B. möglich, durch eine sequentielle DNA-Isolierung, also zunächst nach Zhou et al. und anschließend nach More et al. (1994, Appl. Environ. Microbiol., 60 (5): 1572-1580), selektiv DNA aus Actinomyceten zu isolieren. Hierzu wird zunächst der Zellaufschluß erfindungsgemäß modifiziert nach Zhou et al. durchgeführt, wodurch die DNA der Mikroorganismen mit Ausnahme der Actinomyceten extrahiert wird. Nach einer Inkubation mit SDS folgt ein Zentrifugationsschritt. Die noch nicht aufgeschlossenen Actinomycetenzellen befinden sich nun im Niederschlag (pellet). Nach einem Waschschritt wird die DNA aus diesem Zellpellet mit der Methode nach einem erfindungsgemäß modifizierten Protokoll nach More extrahiert. Hierbei wird z.B. ein Glasperlengemisch mit 0,1 - 0,25 mm Durchmesser verwendet und die Reinigung der DNA mittels Silica vorgenommen, anstatt eine Ethanol-Fällung durchzuführen. Diese erfindungsgemäße Vorgehensweise ist insbesondere vorteilhaft, wenn die anschließende Klonierung der DNA in Streptomyceten, Rhodococcus oder Corynebacterium erfolgen soll.
Ein Vorteil des erfindungsgemäßen Verfahrens ist somit, daß mit dem Überstand und Niederschlag des zuvor genannten Zentrifugationsschrittes getrennte Fraktionen vorliegen, aus denen DNA isoliert werden kann, die überwiegend von Actinomyceten (Niederschlag) oder überwiegend eben nicht aus Actinomyceten (Überstand) stammt.

In einer vorteilhaften Variante der vorliegenden Erfindung werden die aus den Bodenproben extrahierten Nukleinsäuren in Bruchstücke des Größenbereichs von etwa 1-10 kb, bevorzugt von etwa 2-9 kb und besonders bevorzugt von etwa 3-8 kb fragmentiert. Dies erfolgt nach gängigen Methoden beispielsweise in einem partiellen Restriktionsansatz mit den Endonukleasen Sau3AI oder Hsp92II und anschließender Größenfraktionierung über Gelelektrophorese.

In einer Variante des vorliegenden Verfahrens erfolgt die Fragmentierung der Nukleinsäuren unter Zusatz von nicht-acetyliertem Rinderserumalbumin (BSA). Je nach Zusammensetzung der zum Aufschluß eingesetzten Bodenprobe werden u.U. nicht die gesamten Störstoffe, u.a. Huminstoffe, bei der Aufreinigungsprozedur aus der Nukleinsäure-Lösung hinreichend entfernt. Durch den Zusatz von nicht-acetyliertem BSA wird die Hemmung der Restriktionsendonukleasen durch im Nukleinsäureextrakt enthaltene Huminstoffe minimiert. Hierbei ist eine Endkonzentration an nicht-acetyliertem BSA von etwa 1-15 µg/µl, bevorzugt von etwa 2-12 µg/µl und besonders bevorzugt von etwa 10 µg/µl Restriktionsansatz vorteilhaft. Die einzusetzende Menge an nicht-acetyliertem BSA kann darüber hinaus je nach verwendetem Restriktionsenzym (und ggf. Produktionscharge) separat ausgetestet werden und im Einzelfall auch von den oben genannten Werten abweichen.

Das erfindungsgemäße Verfahren zur Herstellung einer normalisierten Genbank zeichnet sich ferner dadurch aus, daß die Quantifizierung der aus Bodenproben extrahierten Nukleinsäuren und/oder deren Fragmente in Schritt c) durch Fluoreszenzfarbstoffe, bevorzugt durch SYBR-Green-I, erfolgt. Dies ist ein besonderer Vorteil des erfindungsgemäßen Verfahrens, da der wässrige Rohextrakt einer aufgeschlossenen Bodenprobe u.a. einen hohen Gehalt an Huminstoffen aufweist, der die photometrische Quantifizierung der DNA in diesem Rohextrakt unmöglich macht, da auch die Huminstöffe im UV-Bereich, z. B. bei 260 nm, stark absorbieren. Das erfindungsgemäße Verfahren löst diese Problematik durch die Quantifizierung der DNA mit Hilfe von Fluoreszenzfarbstoffen, bevorzugt SYBR-Green-I (Molecular Probes, Inc. USA). Allgemein kann es bei Bestimmungen mittels Fluoreszensspektroskopie zur Verfälschung der Ergebnisse aufgrund von Verunreinigungen kommen, hier z. B. Huminstoffe, die zu einem Quenching der Fluoreszenz führen. Vorteil der vorliegenden Erfindung ist, daß sich dieses Quenching durch Verdünnen des Rohextraktes in einer Größenordnung von etwa 1:30 bis 1:50 und gängige Standardadditionsverfahren eliminieren läßt (Skoog, D.A., Leary, J.J.: Instrumentelle Analytik - Grundlagen, Geräte, Anwendungen; S. -176f, 1. Aufl., Springer-Verlag Berlin Heidelberg New York). Der Fehler bei der DNA-Bestimmung aus Bodenproben liegt so erfindungsgemäß nur noch bei etwa 10%.

In einer besonders vorteilhaften Variante der vorliegenden Erfindung werden die fragmentierten Nukleinsäuren mit sogenannten Linkern verknüpft, die wenigstens eine Erkennungsstelle für eine selten vorkommende Restriktionsendonuklease aufweisen. Erfindungsgemäß erfolgt die Ligation der Linker an die fragmentierten Nukleinsäuren, noch bevor die Normalisierung der DNA erfolgt, also vor Schritt d) des oben genannten Verfahrens. Bevorzugt weisen die Linker und bevorzugt auch der beispielsweise zur Klonierung und/oder Amplifikation der isolierten DNA verwendete Vektor eine Genstruktur auf, welche ihrerseits eine Erkennungsstelle für die Restriktionsendonuklease I-Ppol aufweist. Die Endonuklease I-Ppol benötigt eine mindestens 15 Basenpaare (bp) lange Erkennungssequenz. Dies gewährleistet, daß die zur Restriktion eingesetzte Nukleinsäure durch das Enzym nur extrem selten oder gar nicht gespalten wird. So befindet sich im Genom des Bakteriums E. coli keine Erkennungsstelle für I-Ppol und im Genom der Hefe Saccharomyces cerevisiae "schneidet" I-Ppol lediglich dreimal. Erfindungsgemäß denkbar sind im Prinzip jedoch auch andere selten vorkommende Erkennungsstellen für Restriktionsendonukleasen. D.h., alternativ wären auch andere Endonukleasen mit extrem langen Erkennungssequenzen erfindungsgemäß einsetzbar, wie z.B. sogenannte "Homing-Endonukleasen". Ferner ist es erfindungsgemäß auch denkbar, daß die Erkennungsstellen einer Restriktionsendonuklease in den Linkern und im Vektor nicht identisch, aber zumindest kompatibel sind. Gegenstand der vorliegenden Erfindung ist somit auch ein Verfahren, das sich dadurch auszeichnet, daß in Schritt f) ein Vektor eingesetzt wird, der wenigstens eine Erkennungsstelle für eine selten vorkommende Restriktionsendonuklease aufweist, die mit der Erkennungsstelle in den Linkern kompatibel ist. Ein Beispiel erfindungsgemäß bevorzugter Linker ist gemäß SEQ ID Nr. 2 und 3 gezeigt.

Gegenstand der vorliegenden Erfindung ist somit auch eine Genstruktur, enthaltend eine Sequenz gemäß SEQ ID No. 9 mit wenigstens einer multiplen Klonierungsstelle enthaltend wenigstens eine selten vorkommende Erkennungsstelle für das Restriktionsenzym I-Ppol sowie eine Primer-Bindungsstelle und eine T7-Polymeraseerkennungsstelle, die über den Lac-Operator in ihrer Aktivität reguliert wird.

Gegenstand der vorliegenden Erfindung ist ferner die Verwendung der selten vorkommenden Erkennungsstelle für die Restriktionsendonuklease I-Ppol zur Herstellung einer Genstruktur der erfindungsgemäßen Art.

Die vorliegende Erfindung betrifft somit auch einen Vektor, der wenigstens die zuvor charakterisierte Genstruktur sowie zusätzliche Nukleotidsequenzen zur Selektion, zur Replikation in der Wirtszelle und/oder zur Integration in das Wirtszell-Genom aufweist. Beispiele geeigneter Vektoren sind zahlreich aus der Literatur beschrieben, wie z. B. Plasmide der Bluescript-Serie, z. B. pBluescript SK+ (Short, J.M. et al. (1988) Nucleic Acids Res. 16: 7583-7600; Alting-Mees, M.A. and Short, J.M. (1989) Nucleic Acids Res. 17: 9494), pJOE930 (Altenbuchner J. et al (1982) Meth. Enzymol. 216: 457-466), pUC18 oder 19 (Vieira, J. & Messing, J. (1982) Gene 19:259; Yanisch-Perron, C. et al. (1985) Gene 33: 103).

Zur Erstellung einer normalisierten Genbank werden die in der Natur seltener vorkommenden DNA-Spezies angereichert und entsprechend die häufig vorkommenden DNA-Spezies abgereichert. Dies erfolgt im Prinzip durch Denaturierung der aus den Bodenproben isolierten dsDNA und anschließende Renaturierung über einen gewissen Zeitraum, wobei die häufig vorkommenden DNA-Spezies schneller rehybridisieren als die seltenen. Kritisch bei der Normalisierung von DNA ist die Abschätzung des Zeitpunktes zur Beendigung der Renaturierung, um ein optimales Verhältnis zwischen selten vorkommenden und häufig vorkommenden DNA-Spezies zu erzielen, so daß theoretisch alle DNA-Spezies in gleicher Anzahl vorliegen. Wird dieser Schritt der ssDNA/dsDNA-Abtrennung zu früh durchgeführt, ist die Effizienz der Normalisierung nur gering, da immer noch ein Großteil der ssDNA aus gleichartigen, häufig vorkommenden DNA-Molekülen einer Organismenart besteht. Erfolgt andererseits die ssDNA/dsDNA-Abtrennung zu spät, ist womöglich die gesamte ssDNA bereits wieder hybridisiert und liegt doppelsträngig vor. Dies hat den gravierenden Nachteil zur Folge, daß keine ausreichende Menge ssDNA zur Weiterbearbeitung isoliert werden kann und dabei außerdem nicht die gesamte Bandbreite der tatsächlich in der Bodenprobe vorkommenden seltenen DNA-Moleküle vertreten ist.

Es ist daher ein besonderer Vorteil des erfindungsgemäßen Verfahrens, daß der zeitliche Verlauf der Renaturierung der zuvor denaturierten Nukleinsäurefragmente verfolgt werden kann.

Dies erfolgt erfindungsgemäß fluorometrisch mit Hilfe von DNA-spezifischen Fluoreszenzfarbstoffen. Erfindungsgemäß bevorzugt wird hierbei SYBR-Green-I. SYBR-Green-I bietet den Vorteil, qualitativ zwischen ssDNA und dsDNA zu unterschieden. Dies ist aufgrund der unterschiedlichen Fluoreszenzausbeute dieser beiden DNA-Spezies im Komplex mit dem Farbstoff möglich. Der [dsDNA-SYBR-Green-I]-Komplex weist eine wesentlich höhere, z T. bis zu 13-fach höhere, Fluoreszenz auf als der entsprechende ssDNA-Farbkomplex.

Erfindungsgemäß werden während der Rehybridisierung Aliquots aus dem "Normalisierungsansatz" entnommen, mit SYBR-Green-I versetzt und die Fluoreszenz mit der Fluoreszenz des nicht-denaturierten Kontrollansatzes gleicher DNA-Konzentration verglichen. Im Verlauf der Renaturierung nimmt die relative Fluoreszenz aufgrund des steigenden Gehaltes an dsDNA zu. Bei vollständiger Rehybridisierung der ssDNA zu dsDNA wird der ursprüngliche Fluoreszenzwert der nicht-denaturierten Probe erreicht. Problematisch bei der zuvor beschriebenen Vorgehensweise ist die Probennahme bzw. die dabei ggf. auftretende Beeinträchtigung der Hybridisierungsbedingungen und die Zusammensetzung des Rehybridisierungspuffers. Dies wird durch die vorliegende Erfindung in vorteilhafter Weise gelöst, wobei ein nur sehr geringes Probenvolumen von etwa 1-5 µl, bevorzugt 1,5-3 µl, besonders bevorzugt von 1,8 - 2,5 und höchst besonders bevorzugt 2µl für die Fluoreszensspektroskopie ausreicht. Ferner werden die Pipettenspitzen auf eine Temperatur vorgewärmt, die der Hybridisierungstemperatur entspricht, um einer ungenauen Probennahme und einem Abfall der Hybridisierungstemperatur vorzubeugen. In einer erfindungsgemäßen Variante wird ein Hybridisierungspuffer eingesetzt, der maximal 0,01 %, vorzugsweise von 0,0001 bis 0,01%, besonders bevorzugt von 0,0001 bis 0,001 % SDS (v/v) und eine Natriumchlorid-Konzentration zwischen 0,1 M und 1,5 M, bevorzugt von 0,2 M bis 1,0 M, besonders bevorzugt von 0,3 M bis 0,8 M und insbesondere von 0,4 M enthält.

In einer Variante der vorliegenden Erfindung läßt sich aufgrund der dargestellten Vorgehensweise somit beispielsweise bezogen auf eine eingesetzte Konzentration von 1 µg/µl größenfraktionierter DNA (3-6 kb) aus dem Bakterium E. coli ein optimaler Zeitpunkt zum Abbruch der Rehybridisierung im Bereich von etwa 70 - 220 Minuten, bevorzugt von etwa 80 - 200 Minuten und besonders bevorzugt von etwa 100 - 140 Minuten ermitteln.

Nachdem die Denaturierung und anschließende Renaturierung (Rehybridisierung) abgeschlossen ist, wird die noch als Einzelstrang vorliegende DNA (ssDNA) von der renaturierten doppelsträngigen DNA (dsDNA) abgetrennt und beispielsweise mittels PCR amplifiziert. Die mehrfache Wiederholung der zuvor beschriebenen erfindungsgemäßen Schritte der Normalisierung führt zu der gewünschten Anreicherung selten vorkommender DNA-Spezies aus Bodenproben bei gleichzeitiger Abreicherung der häufiger vorkommenden DNA-Spezies, so daß Fraktionen von Nukleinsäure-Spezies erhalten werden, in denen alle DNA-Spezies annähernd gleich häufig (normalisiert) vorliegen.

Für die Abtrennung der ssDNA von der dsDNA stehen grundsätzlich verschiedene Möglichkeiten zur Verfügung, wie beispielsweise Adsorptionschromatographie (z.B. mittels Silicagel oder Hydroxyapatit) oder eine Fragmentierung der dsDNA mittels Restriktionsendonukleasen. Erfindungsgemäß bevorzugt wird ein Verfahren zur Herstellung einer normalisierten Genbank aus Bodenproben, wobei die Adsorptionschromatographie mittels Hydroxyapatit (kristallines Calciumphosphat [Ca₅(PO₄)₃OH]₂) erfolgt. In einer vorteilhaften Variante des erfindungsgemäßen Verfahrens zur Herstellung einer normalisierten Genbank aus Bodenproben erfolgt die Adsorptionschromatographie nicht wie üblich in Form einer Säule, sondern im Batch-Ansatz.
In einer weiteren Variante der vorliegenden Erfindung erfolgt die Adsorptionschromatographie in (Spin)-Säulen (z. B leere Mobicolsäulen der Firma MoBiTec, Göttingen), die mit Hydroxyapatit-Suspension befüllt werden.

Bei der Abtrennung der ssDNA im Batch-Ansatz erfolgt diese erfindungsgemäß durch Zugabe von 10 bis 100 µl Hydroxyapatit-Suspension, bevorzugt 25 - 80 µl und besonders bevorzugt 40 - 60 µl pro 1 µg DNA.
Denkbare Behältnisse, in denen die Abtrennung im Batch-Verfahren erfolgen kann, sind z. B. PCR-Reaktionsgefäße (0,2 ml) oder Standard-Reaktionsgefäße (1,5 bzw. 2 ml).
Um zu erreichen, daß nur die dsDNA an das Hydroxyapatit bindet und die ssDNA im Überstand bleibt, wird erfindungsgemäß der gesamte DNA-Ansatz (Rehybridisierungsansatz) bei Raumtemperatur in ssDNA-Elutionspuffer (Mittelsalzpuffer, z. B. 0,15 - 0,17 M NaPO₄; pH 6,8) aufgenommen und auf das ebenfalls in ssDNA-Elutionspuffer suspendierte Hydroxyapatit aufgegeben.
Die Auftrennung von ssDNA und dsDNA erfolgt erfindungsgemäß bei Temperaturen von 20°C bis 60°C, bevorzugt von 20°C bis 30°C und besonders bevorzugt von 22°C (RT).
Für eine Abtrennung der ssDNA bei RT wird der DNA-Ansatz bei RT in ssDNA-Elutionspuffer 0,17 M NaPO₄ (pH 6,8) aufgenommen. Für eine Abtrennung bei 60°C wird er in 0,15 M ssDNA-Elutionspuffer aufgenommen. Die Elution der gebundenen dsDNA erfolgt mit 0,34 M NaPO₄ (pH 6,8) (dsDNA-Elutionspuffer). Nach Pufferaufgabe und kurzer Zentrifugation befindet sich die jeweils gewünschte DNA im Überstand.
In einer Variante dieses Verfahrens erfolgt die Abtrennung auf Spin-Säulen bei Raumtemperatur. Dabei wird der in ssDNA-Elutionspuffer aufgenommene Rehybridisierungsansatz auf Spin-Säulen gegeben, die mit 50 - 100 µl Hydroxyapatit-Suspension (suspendiert in ssDNA-Elutionspuffer) befüllt sind. Die ssDNA befindet sich nach der Zentrifugation im Eluat; die gebundene dsDNA kann nach Aufgabe von dsDNA-Elutionspuffer ebenfalls durch Zentrifugation eluiert werden.

Die zuvor erläuterte erfindungsgemäße Vorgehensweise im Batch-Ansatz zeichnet sich dabei gegenüber der herkömmlichen Säulenchromatographie durch die Vorteile aus, daß die Bearbeitung einer größeren Anzahl an Proben möglich ist, die Auftrennung konstanter und einfacher temperierbar ist und darüber hinaus schneller erfolgt. Desweiteren ist die Handhabung insgesamt einfacher, als die in den Lehrbüchern, wie z. B. von Maniatis (Maniatis V., Sambrook J, Fritsch EF & Maniatis V (1989). Molecular Cloning: A Laboratory Manual. Vol I-III. Cold Spring Harbour Laboratory Press) beschriebenen Verfahren.

Wie zuvor dargestellt, liegt die gewünschte selten vorkommende ssDNA nach Zentrifugation des Hydroxyapatit-Ansatzes im Überstand bzw. Eluat vor und kann ggf. nach einer Aufreinigung über gängige Verfahren der Gelchromatographie (z. B. Sephadex) oder Butanol-Extraktion zur weiteren Verarbeitung, wie z.B. PCR oder die Klonierung in einen geeigneten Vektor, eingesetzt werden, resultierend in einer normalisierten Genbank aus Nukleinsäuren von im Boden lebenden Mikroorganismen.

Gegenstand der vorliegenden Erfindung ist ferner der Einsatz der erfindungsgemäß hergestellten, normalisierten Genbank zur Identifizierung von Genen kodierend für neue Biokatalysatoren aus im Boden lebenden Mikroorganismen. Entsprechende Vorgehensweisen zur Durchmusterung einer Genbank zur Identifizierung neuer Biokatalysatoren sind dem Fachmann bekannt.

Beispielsweise wird hierzu eine normalisierte Genbank der zuvor beschriebenen Art in einen geeigneten Wirtsorganismus, wie Bakterien und hier z. B. Escherichia coli, Salmonella spec., Streptomyces spec., Streptomyces nidulans, Streptomyces lividans, Bacillus subtilis, Lactococcus oder Corynebacterium oder Hefen, wie z.B. Pichia oder Saccharomyces, übertragen. Die Aufzählung der Wirtsorganismen ist dabei beispielhaft und nicht limitierend für die vorliegende Erfindung. Die erhaltenen transformierten Mikroorganismen werden anschließend zur Selektion neuer Biokatalysatoren auf einem Nährmedium (z.B. LB-Agarplatten) kultiviert, dem denkbare Substrate einer interessanten Enzymklasse, wie beispielsweise Esterasen, Lipasen, Oxygenasen etc., zugesetzt wurden. Als Nährmedien können auch Selektivmedien eingesetzt werden, denen z.B. Giftstoffe zugesetzt sind. Durch das Wachstum der transformierten Mikroorganismen, die Ausbildung eines Lysehofes, die Trübung des Kulturmediums, eine Farbreaktion oder denkbare andere Reaktionen können dann diejenigen Transformanten selektioniert werden, die mit hoher Wahrscheinlichkeit einen neuen Biokatalysator aus bislang im Labor nicht kultivierbaren Mikroorganismen aus Bodenproben enthalten. Aus den ausgewählten Transformanten kann dann die normalisierte DNA (re)-isoliert, sequenziert und weiter charakterisiert werden, resultierend in der Verfügbarkeit entsprechend neuer Gene kodierend für neue Biokatalysatoren mit wirtschaftlich interessantem Anwendungsbereich.

Denkbar ist auch eine Identifizierung der Gene kodierend für neue Biokatalysatoren über Hybridisierungsexperimente der normalisierten Genbank mit geeigneten DNA- oder RNA-Sonden oder Antikörpern.

Die vorliegende Erfindung wird anhand der nachfolgenden Beispiele näher erläutert, die jedoch nicht limitierend für die vorliegende Erfindung sind:

### Beispiele

### 1. Allgemeines

Allgemeine gentechnische bzw. molekulargenetische Vorgehensweisen, wie z. B. Restriktionsansätze, Klonierungen, Anzucht und Selektion transgener Organismen, Agarose-Gelelektrophoresen, Herstellung von Primern, PCR etc. erfolgte nach gängigen Methoden gemäß Maniatis et al. (Maniatis V., Sambrook J, Fritsch EF & Maniatis V (1989). Molecular Cloning: A Laboratory Manual. Vol I-III. Cold Spring Harbour Laboratory Press).

### 2. DNA-Isolierung

### a) DNA-Isolierung modifiziert nach Zhou et al. (1996, Appl. Environ. Microbiol., 62 (2): 316-322) zur Klonierung in E. coli

Mit Hilfe dieser Methode kann DNA aus Bodenproben mit hoher Ausbeute isoliert werden, Actinomyceten jedoch werden kaum aufgeschlossen.

### Puffer:

| | |
|---|---|
| Extraktionspuffer: | 100 mM Tris-HCl, pH 8 100 mM Na-EDTA, pH 8 100 mM Na-Phosphat, pH 8 1,5 M NaCl 1% CTAB (Hexadecyimethylammoniumbromid) |

1g Bodenprobe wird mit 2,6 ml Extraktionspuffer versetzt, gevortext und 3 "freeze-thaw"-Zyklen unterworfen (flüssiger Stickstoff -> +65°C). Danach werden 50 µl Proteinase K (20 mg/ml) zugegeben und unter Schütteln 30 min bei 37°C inkubiert. Anschließend werden 300 µl 20% ige SDS-Lösung zugegeben und 2 Std. bei 65°C inkubiert. Dann wird der Ansatz 10 min bei 5000 rpm zentrifugiert und der Überstand gesammelt. Das Pellet wird 1 mal mit 2 ml Extraktionspuffer und 250 µl 20% SDS gewaschen (10 min Inkubation bei 65°C, Zentrifugation bei 5000 rpm für 10 min). Die vereinigten Überstände werden mit 1/10 Vol. 10% CTAB versetzt, gemischt und 10 min bei 5000 rpm zentrifugiert. Die wäßrige Phase wird mit 1 Vol. Chloroform extrahiert. Dann wird die wäßrige Phase mit 0,7 Vol. Isopropanol gefällt. Das Pellet wird in 100 µl TE aufgenommen.

### b) DNA-Isolierung modifiziert nach More (1994, Appl. Environ. Microbiol., 60 (5): 1572-1580) mit direkter Reinigung

Mit dieser Methode kann DNA aus allen Mikroorganismen, einschl. der Actinomyceten, mit hoher Ausbeute isoliert werden.

### Puffer:

| | |
|---|---|
| Na-Phosphat-Puffer: | 100 mM , pH 8 |
| | |
| 10% SDS-Puffer: | 100 mM NaCl |
| | 500 mM Tris- HCl, pH 8 10% (w/v) SDS |
| | |
| L6-Puffer: | 5 M Guanidin-thiocyanat |
| | 50 mM Tris-HCl, pH8 |
| | 25 mM NaCl |
| | 20 mM EDTA |
| | 1,3% Triton X-100 |
| | |
| L2-Puffer: | 5 M Guanidin-thiocyanat |
| | 50 mM Tris-HCl, pH8 |
| | 25 mM NaCl |
| | |
| Silica: | 4,8 g Silica in 40 ml H₂O suspendieren, 24 h absetzen lassen |
| | 35 ml Überstand abnehmen, mit H₂O auf 40 ml auffüllen, 30 min absetzen lassen |
| | Überstand abnehmen, auf 40 ml auffüllen, ÜN absetzen lassen |
| | 36 ml abnehmen, zum "Pellet" 48 µl 30 %ige HCl geben, vortexen, |
| | aliquotieren, im Dunkeln bei RT lagern |

0,5 g Bodenprobe wird mit 0,5 ml 100 mM Na-Phosphat-Puffer, pH 8, 250 µl SDS-Puffer und 2 g Glasperlen (ø 0,1 mm - 0,25 mm) versetzt und durch Vortexen gemischt. Danach wird der Ansatz 10 min in der Retsch-Mühle mit einer Schüttelfrequenz von 1800 min⁻¹ und einer Amplitude von 80 behandelt und anschließend 10 min bei Raumtemperatur bei 14 000 rpm zentrifugiert. Der Überstand wird abgenommen, das Pellet mit 300 µl Na-Phosphat-Puffer, pH 8 versetzt und 2 min im Ultraschallbad inkubiert und anschließend 3 min bei 14000 rpm abzentrifugiert. Die vereinigten Überstände werden mit 2/5 Vol. 7,5 M NH₄-Acetat versetzt, gevortext und 5 min auf Eis inkubiert. Anschließend wird 3 min bei 14 000 rpm zentrifugiert und der Überstand (650 µl) mit 1/10 Vol. Silica (65 µl) und 2 Vol. Puffer L6 (1,3 ml) versetzt, gemischt und 3 min bei 3000 rpm zentrifugiert. Der Überstand wird abgekippt, 1,3 ml Puffer L2 zugegeben und durch Schütteln gut gemischt. Dann wird 3 min bei 3000 rpm zentrifugiert, der Überstand verworfen und das Pellet mit 1,5 ml 70% Ethanol gewaschen (schütteln, 3 min bei 3000 rpm zentrifugieren) und getrocknet. Zur Elution der DNA werden 100 ml TE zugegeben, 10 min bei 56°C unter Schütteln inkubiert und 1 min bei 14000 rpm abzentrifugiert, der Überstand wird vorsichtig abgenommen und in ein frisches Eppendorfgefäß überführt.

### 3. Aufreinigung der DNA

Hierzu werden folgende Materialien verwendet:
Ausschlußchromatographie-Säulen: CHROMA SPIN^{™}-1000 Column, CLONTECH Laboratories, Inc.; Elutionspuffer: 10 mM Tris/HCl pH 8,5. Bevor die isolierte Boden-DNA auf die CHROMA SPIN^{™}-1000-Säule aufgegeben wird, wird die Säule mit dem Elutionspuffer (10 mM Tris/HCl [pH 8,5]) nach Herstellerangaben gespült.
Bis zu 100 µl der isolierten Boden-DNA werden auf die CHROMA SPIN-1000-Säule aufgegeben und nach Herstellerangaben eluiert. Der Grad der Aufreinigung kann dadurch abgeschätzt werden, daß vor und nach dem Reinigungsschritt UV/VIS-Spektren aufgenommen werden. Eine Abnahme der Absorption über den gesamten UV/VIS-Bereich deutet auf eine Verminderung der Huminstoffkonzentration in der Probelösung hin (Die Absorptionsbande der Nukleinsäuren liegt zwischen ca. 230 und 300 nm).

### 4. Quantifizierung der DNA durch Fluoreszenzfarbstoffe

Es wurden folgende Materialien verwendet:
- TE-Puffer: 10 mM Tris/HCl [pH 7,5], 1 mM EDTA [pH8,0]
- SYBR Green I: Sigma, Arbeitslösung: 1:3750 (mit TE verdünnt)
- Kalbsthymus-DNA: Sigma, Stammlösung und Standards mit TE-Puffer angesetzt
   - Stammlösung:: 100 µg/ml
   - Standards:: 0,0 µg/ml; 0,3 µg/ml; 0,7 µg/ml; 1,0 µg/ml; 2,0 µg/ml; 3,0 µg/ml; 4,0 µg/ml; 5,0 µg/ml
- Fluorimeter: Excitationsswellenlänge: 485 nm Emissionswellenlänge: 535 nm (optimal: 524 nm)

### Vorversuch:

Der aufgeschlossene Rohextrakt wird zunächst so stark verdünnt (z.B. 1:50, letztendlich abhängig vom dsDNA-Gehalt in der Bodenprobe und vom Aufschlußverfahren), so daß einerseits die Absorption bei 535 nm und 485 nm ≤ 0,05 liegt, gleichzeitig aber noch genügend dsDNA in der verdünnten Probe vorhanden ist, um eine exakte Messung zu gewährleisten. Dazu wird ein Vorversuch durchgeführt, bei dem die Fluoreszenzen unterschiedlicher Verdünnungsstufen des Rohextraktes ermittelt werden. Diese Fluoreszenzwerte müssen natürlich innerhalb der verwendeten Kalibriergerade liegen und mindestens das 5-6fache der Fluoreszenz des Kalibriergeradenblindwertes betragen.

### Standardaddition: (Zugabe der DNA-Standards zu Aliquots der verdünnten Probe)

50 µl verdünnte Probe (siehe 1.)
+ 50 µl DNA-Standard (0,0 - 5 µg/ml; Kalbsthymus DNA)
+ 150 µl SYBR Green I (1:3750 mit TE, pH 7,5)

### Kalibriergerade:

50 µl TE
50 µl DNA-Standard (0,3 - 5 µg/ml; Kalbsthymus DNA)
+ 150 µl SYBR Green I (1:3750 mit TE, pH 7,5)

Dotierung des Rohextraktes mit Kalbsthymus-DNA zur Ermittlung der Wiederfindung:
Ein Aliquot des Rohextraktes wird mit einer DNA-Lösung bekannter Konzentration dotiert. Beispiel: 300 µl aufgeschlossener Rohextrakt wird mit 5 µl Kalbsthymus-DNA (100 µg/ml) versetzt. Dieses dotierte Aliquot wird in der gleichen Weise wie die Probe unter 2. verdünnt und weiter nach dem Standardadditionsverfahren die dsDNA-Konzentration bestimmt (siehe 2. (statt "verdünnte Probe" jetzt "dotierte Probe" einsetzten)).

### Reaktionsbedingungen/Meßparameter:

Reaktionsdauer: 10 min (im Dunkeln)
Temperatur: Raumtemperatur
Anregungswellenlänge: 485 nm
Emissionswellenlänge: 535 nm
Standardmikrotiterplatten (optimal mit schwarzen Wells)

### Auswertung (beispielhaft):

- Im Vorversuch wurde eine Verdünnung des Rohextraktes von 1:30 als ausreichend ermittelt (Abs(535nm) = 0,008; Abs(485nm) = 0,021; )
- Berechnung des Steigungskorrekturfaktor K:
   - a) aus den o.g. Angaben "Kalibriergerade" berechnet sich die Steigung der Kalibriergeraden m(Kalib)
      b) aus den o.g. Angaben "Standardaddition" berechnet sich die Steigung der Kalibriergeraden m(Probe) bzw. m(dotierte Probe)
      c) Steigungskorrekturfaktor K = m(Kalib) / m(Probe) (bzw. m(dotierte Probe))
      d) nun werden die Fluoreszenzwerte F aus der Standardaddition der Probe bzw. der dotierten Probe mit dem Steigungskorrekturfaktor K multipliziert ⇒ Fₖₒᵣᵣ.
      e) mit den korregierten Fluoreszenzwerten Fₖₒᵣᵣ. wird die dsDNA-Konzentration in der Probe bzw. in der dotierten Probe nach der üblichen Auswertungsmethode für Standardaddtionsverfahren ermittelt.

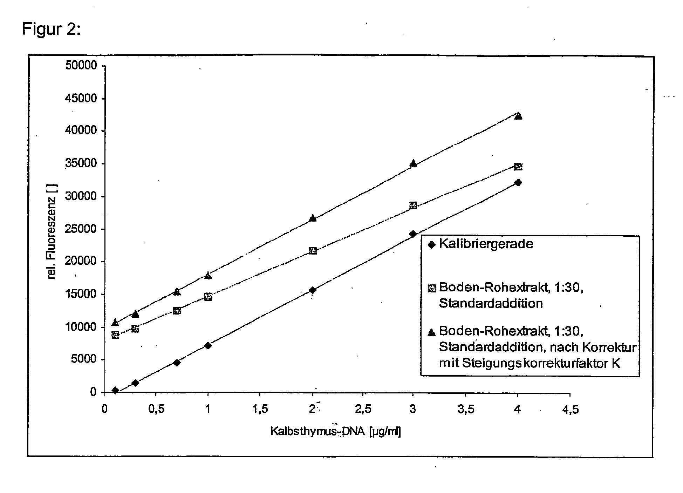

Ein Beispiel für die Auswertung eines Boden-Rohextraktes ist in Tabelle 1 und Figur 2 wiedergeben.

### 5. Fragmentierung der DNA

Die Fragmentierung der DNA erfolgt nach gängiger Laborpraxis. Im einzelnen wird hier die genomische DNA mit *Hsp*92II (Promega) unter Zugabe von 10 µg/µl nichtacetyliertem BSA (DNAse-frei, von Sigma) bei 37 °C verdaut. Die Reaktion wird durch Zugabe von 1/10 Vol. EDTA (0,5 M, pH 8,0) gestoppt. Die genau benötigten Reaktionszeiten, Enzym- und BSA-Mengen für einen limitierten Verdau hängen stark von den DNA-Chargen ab und müssen daher in Vorversuchen eigens bestimmt werden. Die entsprechenden Vorgehensweisen sind dem Fachmann vertraut. Die verdaute DNA wird mit Isopropanol gefällt, in H₂O aufgenommen und über ein 0,8%iges Agarose-Gel aufgetrennt. Der Größenbereich 3 - 5 kb wird mit Hilfe von QIAquick-Säulen (Qiagen) aus dem Gel aufgereinigt.

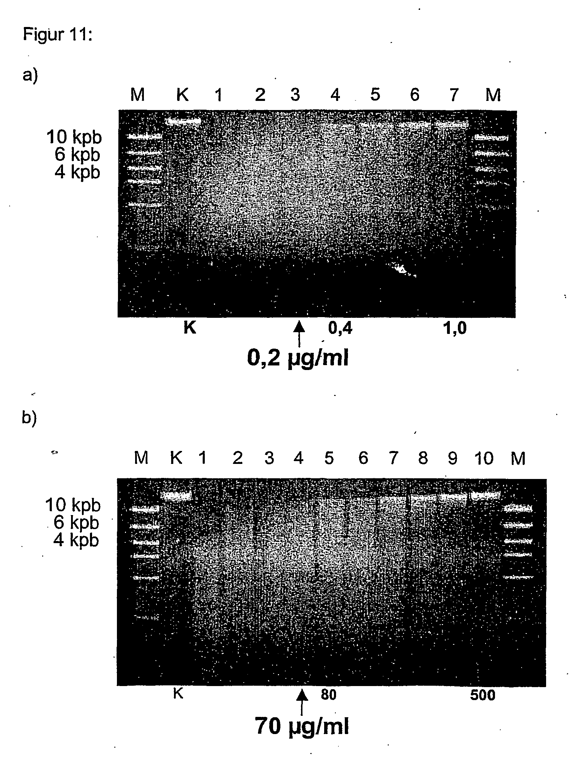

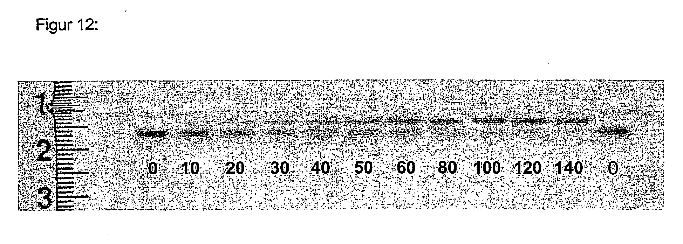

Der positive Effekt von nicht-acetyliertem BSA hinsichtlich der Restriktionsendonukleasen wurde durch differenzspektroskopische Untersuchungen und mit Hilfe von Bandshift-Versuchen untersucht. Dabei konnte gezeigt werden, daß es zu einer Wechselwirkung zwischen dem nicht-acetylierten BSA und den Huminsäuren kommt (käufliche Huminsäuren (Fluka) verwendet) (Figur 11). Die Zugabe von nicht-acetyliertem BSA zum Reaktionsansatz ermöglicht den Verdau von genomischer DNA in Gegenwart von höheren Konzentrationen an Huminstoffen im Vergleich zu Durchführung der Reaktion ohne BSA-Zugabe. Im Fall von der Restriktionsendonuklease Sau 3Al kann die Konzentration an Huminsäuren ca. 350-fach höher sein, wenn nichtacetyliertes BSA zur Reaktion zugefügt wird (minimal-inhibierende-Konzentration [MIC] der Huminsäure ohne BSA-Zugabe: ca. 0,2 µg/ml, MIC der Huminsäure mit BSA-Zugabe: ca. 70 µg/ml; beispielhaft an käuflichen Huminsäuren (Fa. Fluka, Lot 45729/1) ermittelt) (Figur 12). Die Restriktionsendonukleasen reagieren allerdings unterschiedlich empfindlich bezüglich den Huminstoffen und haben somit auch unterschiedliche MIC. Für das Enzym *Hsp* 92II wurde eine MIC der Huminstoffe ohne BSA-Zugabe von ca. 0,2 µg/ml festgestellt. Durch Zugabe von nicht-acetyliertem BSA erhöhte sich die MIC auf ca. 3,0 µg/ml Huminstoffe (Faktor: 15). Ebenso muß die optimale BSA-Konzentration für jedes Restriktionsenzym ermittelt werden. Bei *Sau* 3AI liegt diese bei 8 µg/µl Reaktionslösung (final), für *Hsp* 92II wurde eine optimale BSA-Konzentration von 2 µg/µl Reaktionsansatz festgestellt. Eine weitere Erhöhung der BSA-Konzentration hatte keinen positiven Effekt auf die MIC.
Um Optimierungsschritte zu sparen, wird allgemein eine finale BSA-Konzentration von 10 µg/µl empfohlen: Nach der Zugabe von nicht-acetyliertem BSA zum Reaktionsansatz (Enzym nicht noch zugegeben) sollte eine Präinkubationszeit von 5 min eingehalten werden. Durch diesen Schritt soll gewährleistet werden, daß das nicht-acetylierte BSA genügend Zeit hat, um mit den Huminstoffen vollständig abzureagieren.

### 6. Ligation mit zur Klonierung und Amplifizierung geeigneten Linkern

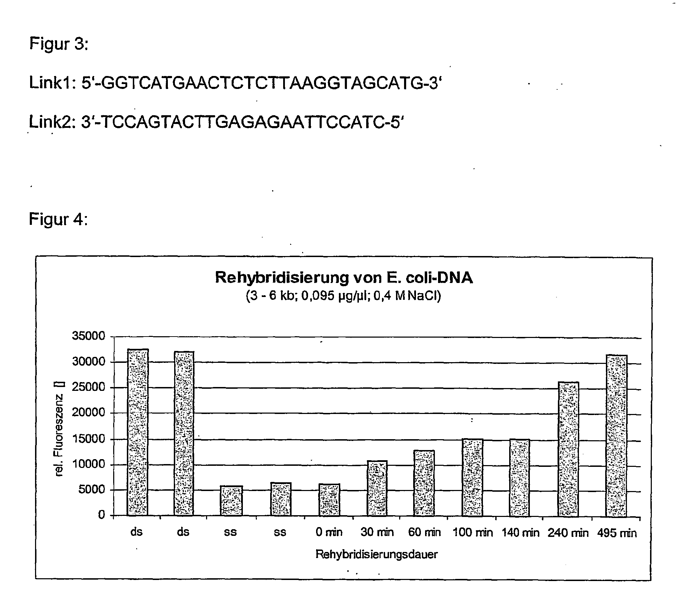

Geeignete Linker sind in Figur 3 gemäß SEQ ID No. 1 und 2 dargestellt. Die Ligation der Linker mit der fragmentierten DNA aus Bodenbakterien erfolgt nach Herstellerangaben (LigaFast Rapid DNA Ligation System von Promega).

### 7. Normalisierung der DNA

Hierzu wurden folgende Materialien verwendet:
- TE-Puffer: 10 mM Tris/HCl [pH 7,5], 1 mM EDTA [pH 8,0]
- SYBR Green I: Sigma, Arbeitslösung: 1:4000 mit TE-Puffer verdünnt
- 3 M NaCl- Lösung
- Hamstofflösungen: 1 M; 2 M
- fragmentierte DNA (3-10 kbp, partieller Verdau): 0,1 µg/µl (im Reaktionsgefäß)
- 200 µl Reaktionsgefäße
- Pipettenspitzen auf 65 °C vorwärmen
- Fluorimeter: Anregungswellenlänge. 485 nm Emissionswellenlänge: 535 nm (optimal: 524 nm)
- Thermocycler mit beheizbarem Deckel

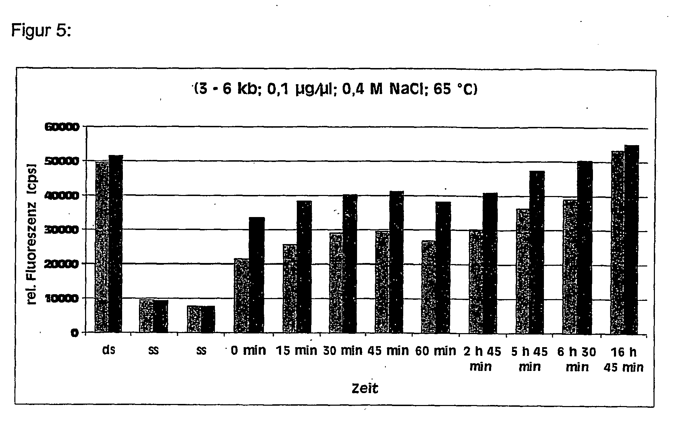

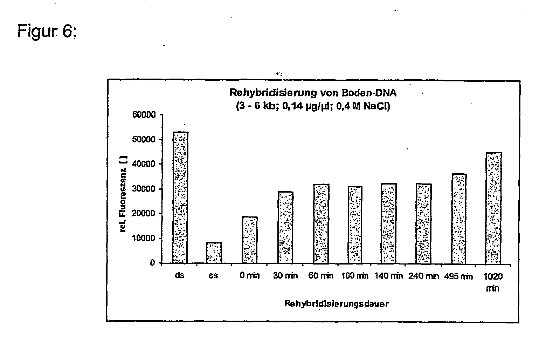

Die zu normalisierende Probe (30 µl Volumen; 0,1 µg/µl DNA (3-10 kbp), 0,4 M NaCl) wird zunächst auf 65 °C erwärmt. Anschließend wurde ein Aliquot von 2 µl entnommen und in 18 µl 1 M Harnstofflösung überführt. Diese Lösung wird sofort auf Eis gelagert (= N_{dsDNA}). Die Denaturierung der Probe erfolgt 95 °C für 5 min. Nach dem Abkühlen der Probe auf die Rehybridisierungstemperatur (65 °C) wird wieder ein Aliquot von 2 µl entnommen, in 18 µl 1 M Harnstofflösung überführt (= N₀) und sofort auf Eis gelagert. Im Verlauf der gesamten Rehybridisierungsdauer werden zu verschiedenen Zeitpunkten weitere Aliquots entnommen. Die Fluoreszenz-Messung erfolgt wie nachfolgend beschrieben:
20 µl Probe (N_{dsDNA}, No, N₁ₕ, ...)
+ 100 µl 2 M Harnstofflösung
+ 80 µl SYBR Green I (1:4000 in TE)
werden in Standardmikrotiterplatten (optimal mit schwarzen Wells) angesetzt und 10 min im Dunkeln bei Raumtemperatur inkubiert; Anregungswellenlänge: 485 nm; Emissionswellenlänge: 535 nm. Die Auswertung erfolgt durch Auftragung der relativen Fluoreszenz gegen die Renaturierungsdauer. Das Ergebnis ist als Balkendiagramm in den Figuren 4, 5 und 6 dargestellt.

### 8. Auftrennung der ssDNA über Hydroxyapatit

a) Abtrennung der ssDNA bei Raumtemperatur im Batch-Verfahren Chemikalien und Geräte
   - Hydroxyapatit: Bio-Gel HTP Hydroxyapatite bzw. DNA Grade Bio-Gel HTP Hydroxyapatite (Biorad)
   - ssDNA-Elutionspuffer: 0,17 M NaPO₄ [pH 6,8]
   - dsDNA-Elutionspuffer: 0,34 M NaPO₄ [pH 6,8] Durchführung
      Nach der Normalisierung kühlt die DNA-Lösung 5 min bei Raumtemperatur ab. Falls pH und Phosphatkonzentration des Rehybridisierungspuffers nicht den Bedingungen des ssDNA-Elutionspuffers entsprechen, wird die DNA-Lösung auf ssDNA-Elutionspuffer-Bedingungen (0,17 M NaPO₄ [pH 6,8]) durch Zugabe von höherkonzentriertem NaPO₄-Puffer eingestellt. Zur Bindung an das Hydroxyapatit werden der DNA-Lösung z. B. 50 µl einer Hydroxyapatit-Suspension (in ssDNA-Elutionspuffer) zugegeben, kurz gemischt (Vortex), 1 min bei RT inkubiert, erneut gemischt und 1 min bei RT inkubiert. Nach anschließender Zentrifugation (2-5 sec, RT), wird der Überstand abgenommen, dieser enthält den Großteil der ssDNA.
      Zur Elution der restlichen ssDNA werden 30 µl ssDNA-Elutionspuffer zugegeben, gemischt, 2-5 sec zentrifugiert und der Überstand abgenommen. Diese Prozedur wird mindestens 5 mal wiederholt.
b) Abtrennung der ssDNA bei Raumtemperatur mit Hydroxyapatit als Spin-Säule
   Chemikalien und Geräte
   - Wie unter 8a)
   - leere Mobicoi-Säulen mit kleinen Filtern (Durchmesser 2,7 mm, 35 µM Porengröße) der Firma MoBiTec (Göttingen).
      Durchführung
      Die Denaturierung, Renaturierung, das Abkühlen auf RT sowie die Einstellung auf ssDNA-Elutionspuffer-Bedingungen erfolgen wie unter (8a) angegeben.
      Zur Herstellung der Hydroxyapatit-Spin-Säule werden 50 µl einer Hydroxyapatit-Suspension (in ssDNA-Elutionspuffer) in die Mobicol-Säule pipettiert und kurz abzentrifugiert. Die DNA-Lösung wird auf die Hydroxyapatit-Spin-Säule aufgetragen und DNA und Hydroxyapatit werden vorsichtig gemischt. Nach kurzer Zentrifugation bei RT enthält das Eluat den Großteil der ssDNA. Die restliche ssDNA kann durch Elution mit 150 µl ssDNA-Elutionspuffer gewonnen werden.

### 9. Amplifikation der ssDNA zur Klonierung in geeignete Genkonstrukte oder Vektoren

Die Amplifikation der ssDNA erfolgt mit dem Expand Long Template PCR System von Roche nach Herstellerangaben. Als Primer dient das auch im Linker benutzte Oligonukleotid Link1. Die Abtrennung unspezifischer PCR-Produkte und der während der PCR .nicht benötigten Primer erfolgt über ein Agarose-Gel (0,8 % Agarose). Der Größenbereich 3 - 5 kb wird mit Hilfe von QIAquick-Säulen (Qiagen) eluiert. Die PCR-Fragmente werden mit I-*Pp*ol verdaut und über QIAquick-Säulen (Qiagen) aufgereinigt. Das Eluat wird zur Ligation mit entsprechend vorbehandelten Genkonstrukt pSCR, das mit I-*Pp*ol (Promega) linearisiert und mit CIAP (Promega) dephosphoryliert wurde, eingesetzt. Anschließend wird *E. coli* BL21 (DE3) pLysS mit dem Konstrukt transformiert.

### 10. Herstellung des Genkonstrukts pSCR

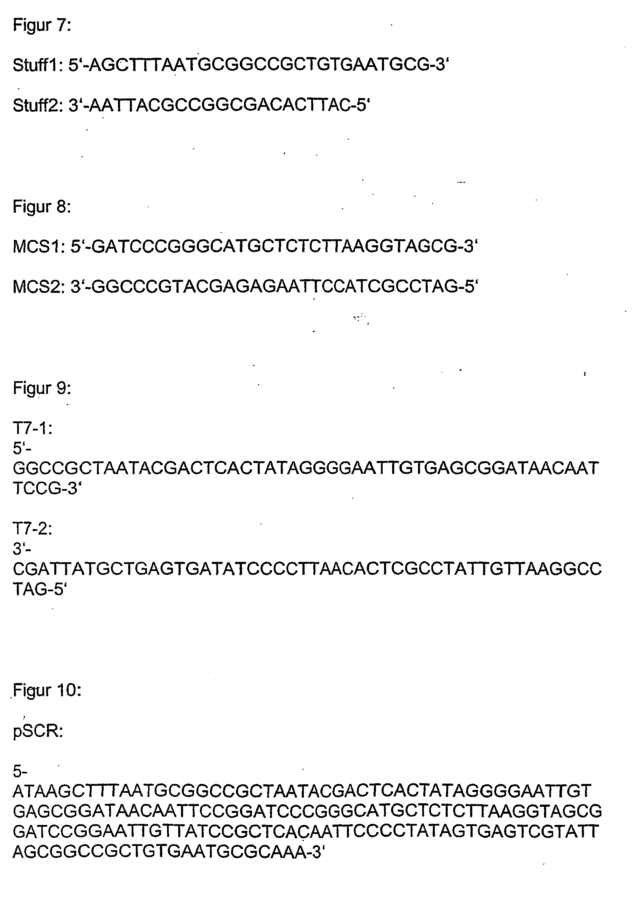

Zur Herstellung des pSCR-Plasmids wurde das Plasmid pBR322 mit *Hin*dIII (NEB) und *Mva*1269I (Fermentas) verdaut und ein 3033 bp langes Fragment isoliert.
In diesen Vektor wurde zunächst der Stuffer stuff1 bzw. stuff2 gemäß SEQ ID No. 3 und 4 ligiert. Das resultierende Plasmid wurde mit *Not*I geschnitten und die Promotorregionen T71 bzw. T72 (aus dem Plasmid pET-15b, Promega) ligiert, was in zwei einander gegenüber orientierten T7-Promotoren resultierte. In die dazwischen liegende *Bam*HI-Schnittstelle wurden dann die Oligonukleotide für die multiplen Klonierstellen MCS1 bzw. MCS2 gemäß SEQ ID No. 5 und 6 ligiert, die eine 1-Ppol-Schnittstelle aufweisen. Die entsprechenden Sequenzabschnitte und die Gesamtsequenz des Genkonstruktes pSCR sind in den Figuren 7-9 bzw. Figur 10 dargestellt.

### 11.Screening der normalisierten Genbank zur Identifizierung von Genen kodierend für neue Biokatalysatoren aus Bodenproben

Nach der Transformation erfolgt das Screening, beispielsweise auf Esterasen, indem die frisch transformierten Zellen direkt auf (trübe) Tributyrin-Platten (1,5% Agar, 1 % (w/v) Tributyrin, LB-Medium; vor dem Autoklavieren homogenisieren) ausplattiert werden. Nach Inkubation für 24 Stunden bei 37 °C werden die trüben Platten bei 4 °C gelagert und jeden Tag zur Überprüfung auf Bildung eines klaren (Lyse)-Hofes untersucht. Klone, um die sich ein Lysehof gebildet hat werden als potentiell Esterase-positiv identifiziert. Diese Klone werden von der Selektivmediumplatte in Vollmedium überführt, angezüchtet und weiteren Analysen unterzogen.

Legende zu den Tabellen und Figuren:
- Tabelle 1:: Beispiel für die Auswertung der fluorimetrischen Quantifizierung von dsDNA aus aufgeschlossenen. Bodenproben ohne vorherige Aufreinigung.

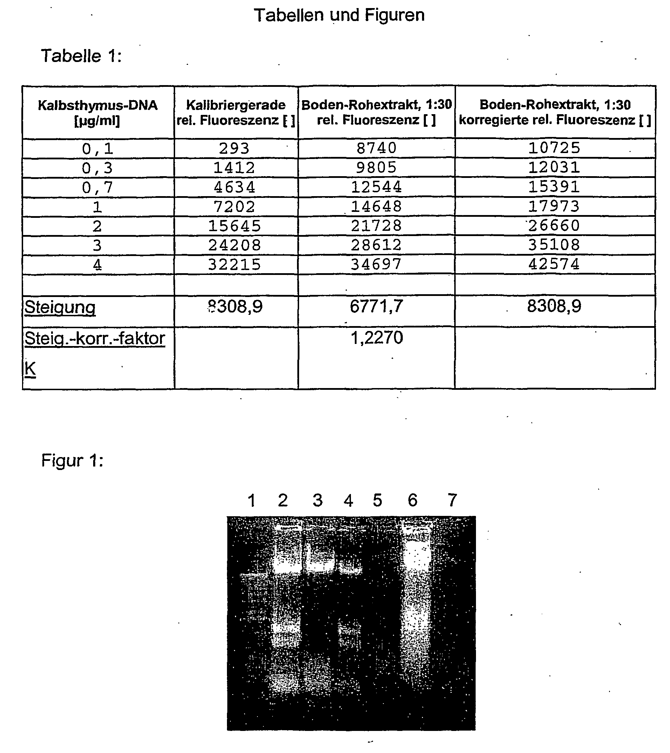

- Figur 1:: Gelelektrophoretische Auftrennung von DNA isoliert aus Bodenbakterien. Spur 1: Größenmarker (kb), Spur 2: Arthrobacter-More, Spur 3: Pseudomonas - More, Spur 4: Rhodococcus - More, Spur 5: Arthrobacter- Zhou, Spur 6: Pseudomonas - Zhou, Spur 7: Rhodococcus - Zhou.
- Figur 2:: Graphische Darstellung des Korrekturverfahrens zur fluorimetrischen Quantifizierung von dsDNA in aufgeschlossenen Bodenproben.
- Figur 3:: Nukleotidsequenzen gemäß SEQ ID No. 1 und 2 entsprechend den bevorzugt verwendeten Linkern link1 und link2 zur Herstellung eines bevorzugten Genkonstruktes.
- Figur 4:: Darstellung der Rehybridisierung von E. coli-DNA durch die Auftragung der relativen Fluoreszenz im Verhältnis zur Rehybridisierungsdauer.
- Figur 5:: Darstellung der Rehybridisierung von Pseudomonas-DNA sowie einem Gemisch aus Pseudomonas und E. coli-DNA im Verhältnis 2:1 durch die Auftragung der relativen Fluoreszenz im Verhältnis zur Rehybridisierungsdauer.
- Figur 6:: Darstellung der Rehybridisierung von Bodenproben-DNA durch die Auftragung der relativen Fluoreszenz im Verhältnis zur Rehybridisierungsdauer.
- Figur 7:: Nukleotidsequenzen gemäß SEQ ID No. 3 und 4 entsprechend den bevorzugt verwendeten stuffern stuff1 und stuff2 zur Herstellung eines bevorzugten Genkonstruktes.
- Figur 8:: Nukleotidsequenzen gemäß SEQ ID No. 5 und 6 entsprechend den bevorzugt verwendeten multiplen Klonierstellen MCS1 und MCS2 zur Herstellung eines bevorzugten Genkonstruktes.
- Figur 9:: Nukleotidsequenzen gemäß SEQ ID No. 7 und 8 entsprechend den bevorzugt verwendeten T7-Promotoren T7-1 und T7-2 zur Herstellung eines bevorzugten Genkonstruktes.
- Figur 10:: Nukleotidsequenz gemäß SEQ ID Nr. 9 einer bevorzugten Genstruktur pSCR enthaltend stuffer-Sequenzen stuff 1. und stuff 2, zwei entgegengesetzte Promotorsequenzen des T7-Promotors aus dem Plasmid pET-15b von Promega, der durch den lac-Operator reguliert wird sowie multiple Klonierungsstellen enthaltend wenigstens die selten vorkommende Erkennungssequenz für die Restriktionsendonuklease I-Ppol aus Physarum polycephalum.
- Figur 11:: Minimal inhibierende Konzentration (MIC) von Huminsäuren (Fluka) für das Restriktionsenzym Sau3Al ohne (a) bzw. mit (b) Zugabe von nicht-acetyliertem BSA. Verdaut wurden 1 µg genomische E. coli-DNA mit Sau3AI (0,3 µg, absolut) in Anwesenheit von steigenden Konzentrationen an Huminsäuren.
a) Spur M: Größenmarker; Spur K: ohne Sau3Al; Spur 1-7: 0; 0,1; 0,2; 0,4; 0,6; 0,8; 1,0 µg/ml Huminsäuren.
b) Spur M: Größenmarker; Spur K: ohne Sau3Al; Spur 1-10: 0; 50; 60; 70; 80; 90; 100; 150; 200; 500 µg/ml Huminsäuren.
Ohne die Anwesenheit von nicht-acetyliertem BSA (a) wurde die DNA in Gegenwart von 0,2 µg/ml Huminsäuren noch verdaut. Bei höheren Huminsäure-Konzentrationen wurde das Enzym sehr stark inhibiert. Bei Zugabe von 10 µg/µl (final) an nicht-actetyliertem BSA zum Reaktionsanatz wurde die genomische DNA noch in Gegenwart von 70 µg/ml Huminsäuren vollständig verdaut.
- Figur 12:: Bandshift-Assay zum Nachweis der Wechselwirkung von Huminsäuren (Fluka) und nicht-acetyliertem BSA. 20 µg Huminsäuren wurden mit steigenden Gehalten an nicht-acetyliertem BSA inkubiert und elektrophoretisch analysiert (1,0 %iges Agarosegel). In Gegenwart von nicht-acetyliertem BSA tritt eine zusätzliche Bande auf, die in der Kontrollbande (0 µg BSA) nicht nachzuweisen ist.

## Patentansprüche

1. Verfahren zur Herstellung einer normalisierten Genbank aus Nukleinsäure-Extrakten von Bodenproben, wobei
a) Nukleinsäuren aus in Bodenproben enthaltenen Lebewesen extrahiert werden,
b) eine Fragmentierung der Nukleinsäuren erfolgt,
c) eine Quantifizierung der Nukleinsäurefragmente mittels Fluoreszenzfarbstoffen erfolgt,
d) eine Normalisierung der Nukleinsäurefragmente erfolgt, wobei die Nukleinsäurefragmente zunächst denaturiert werden und der Verlauf der Renaturierung mittels Fluoreszenzfarbstoffen verfolgt wird,
e) nach Beendigung der Renaturierung eine Trennung der doppelsträngig vorliegenden Nukleinsäuren von den einzelsträngig vorliegenden Nukleinsäuren durch Adsorptionschromatographie erfolgt, wobei die in der Fraktion der einzelsträngigen Nukleinsäuren enthaltenen Nukleinsäure-Spezies annähernd gleich häufig (normalisiert) vorliegen,
f) zur Erstellung der Genbank die normalisierten Nukleinsäure-Spezies in einen Vektor kloniert werden.

2. Verfahren gemäß Anspruch 1, **dadurch gekennzeichnet, daß** eine selektive Isolierung von Nukleinsäuren aus Actinomyceten erfolgt.

3. Verfahren gemäß einem der Ansprüche 1 bis 2, **dadurch gekennzeichnet, daß** bei der Fragmentierung der Nukleinsäuren nichtacetyliertes Rinderserumalbumin in Konzentrationen von etwa 1-15 µg/pl, bevorzugt von etwa 2-12 µg/µl und besonders bevorzugt von etwa 10 µg/µl Restriktionsansatz eingesetzt wird.

4. Verfahren gemäß einem der Ansprüche 1 bis 3, **dadurch gekennzeichnet, daß** die fragmentierten Nukleinsäuren mit Linkern verknüpft werden, die wenigstens eine Erkennungsstelle für eine selten vorkommende Restriktionsendonuklease aufweisen.

5. Verfahren gemäß einem der Ansprüche 1 bis 4, **dadurch gekennzeichnet, daß** die Linker eingesetzt werden, die eine Erkennungsstelle für das Restriktionsenzym I-*Pp*ol aufweisen.

6. Verfahren gemäß einem der Ansprüche 1 bis 5, **dadurch gekennzeichnet, daß** in Schritt f) ein Vektor eingesetzt wird, der wenigstens eine Erkennungsstelle für eine selten vorkommende Restriktionsendonuklease aufweist, die mit der Erkennungsstelle in den Linkern kompatibel ist.

7. Verfahren gemäß einem der Ansprüche 1 bis 6, **dadurch gekennzeichnet, daß** die Quantifizierung der aus Bodenproben extrahierten Nukleinsäuren und/oder deren Fragmente in Schritt c) durch Fluoreszenzfarbstoffe, bevorzugt durch SYBR-Green-I, erfolgt.

8. Verfahren gemäß einem der Ansprüche 1 bis 7, **dadurch gekennzeichnet, daß** der zeitliche Verlauf der Renaturierung der zuvor denaturierten Nukleinsäurefragmente in Schritt d) fluorimetrisch mit DNA-spezifischen Fluoreszenzfarbstoffen, bevorzugt mit SYBR-Green-I, verfolgt wird.

9. Verfahren gemäß einem der Ansprüche 1 bis 8, **dadurch gekennzeichnet, daß** die Adsorptionschromatographie in Schritt e) mittels Hydroxyapatit erfolgt.

10. Verfahren gemäß einem der Ansprüche 1 bis 9, **dadurch gekennzeichnet, daß** die Adorptionschromatographie in Schritt e) im Batch-Ansatz erfolgt.

11. Verfahren gemäß einem der Ansprüche 1 bis 10, **dadurch gekennzeichnet, daß** die Auftrennung von ssDNA und dsDNA bei 20 bis 60°C, bevorzugt bei 20 bis 30°C und besonders bevorzugt bei 22°C durchgeführt wird.

12. Verfahren gemäß einem der Ansprüche 1 bis 11, **dadurch gekennzeichnet, daß** die Auftrennung von ssDNA und dsDNA in einem NaPO₄-Puffer mit einer Konzentration von 0,15 - 0,17 M durchgeführt wird.

13. Verfahren gemäß einem der Ansprüche 1 bis 12, **dadurch gekennzeichnet, daß** die Adsorptionschromatographie in Schritt e) in Spin-Säulen erfolgt.

14. Genstruktur enthaltend eine Sequenz gemäß SEQ ID No. 9 mit wenigstens einer multiplen Klonierungsstelle enthaltend wenigstens eine selten vorkommende Erkennungsstelle für das Restriktionsenzym I-Ppol sowie eine Primer-Bindungsstelle und eine T7-Polymeraseerkennungsstelle, die über den Lac-Operator in ihrer Aktivität reguliert wird.

15. Vektor enthaltend wenigstens eine Genstruktur gemäß Anspruch 14.

16. Verwendung der selten vorkommenden Erkennungsstelle für die Restriktionsendonuklease I-Ppol zur Herstellung einer Genstruktur gemäß Anspruch 14 oder einen Vektor gemäß Anspruch 15.

17. Verwendung der normalisierten Genbank hergestellt nach einem Verfahren gemäß einem der Ansprüche 1-13 zur Selektion von Genen kodierend für Biokatalysatoren aus im Boden lebenden Mikroorganismen.

## Claims

1. A method for preparing a normalized gene bank from nucleic acid extracts of soil samples, which method comprises
a) extracting nucleic acids from living organisms present in soil samples,
b) fragmenting said nucleic acids,
c) quantifying the nucleic acid fragments by means of fluorescent dyes,
d) normalizing said nucleic acid fragments, first denaturing the latter and then monitoring the course of renaturation by means of fluorescent dyes,
e) separating, after renaturation has ended, the double-stranded nucleic acids from the single-stranded nucleic acids by adsorption chromatography, the amount of nucleic acid species present in the fraction of the single-stranded nucleic acids being frequently approximately equal (normalized),
f) generating the gene bank by cloning the normalized nucleic acid species into a vector.

2. The method according to claim 1, wherein nucleic acids are selectively isolated from actinomycetes.

3. The method according to either of claims 1 and 2, wherein nonacetylated bovine serum albumin at concentrations of about 1-15 µg, preferably of about 2-12 µg, and particularly preferably of about 10 µg, per µl of restriction mixture is used in fragmentation of the nucleic acids.

4. The method according to any of claims 1 to 3, wherein the fragmented nucleic acids are linked to linkers which have at least one recognition site for a rarely occurring restriction endonuclease.

5. The method according to any of claims 1 to 4, wherein the linkers which have a recognition site for the restriction enzyme I-Ppol are used.

6. The method according to any of claims 1 to 5, wherein step f) employs a vector which has at least one recognition site for a rarely occurring restriction endonuclease which is compatible with the recognition site in the linkers.

7. The method according to any of claims 1 to 6, wherein the nucleic acids extracted from soil samples and/or their fragments are quantified in step c) using fluorescent dyes, preferably SYBR-Green-I.

8. The method according to any of claims 1 to 7, wherein the time course of renaturation of the previously denatured nucleic acid fragments in step d) is fluormetrically monitored using DNA-specific fluorescent dyes, preferably SYBR-Green-I.

9. The method according to any of claims 1 to 8, wherein adsorption chromatography in step e) is carried out by means of hydroxyapatite.

10. The method according to any of claims 1 to 9, wherein the adsorption chromatography in step e) is carried out in a batch process.

11. The method according to any of claims 1 to 10, wherein ssDNA and dsDNA are fractionated at from 20 to 60°C, preferably at from 20 to 30°C, and particularly preferably at 22°C.

12. The method according to any of claims 1 to 11, wherein ssDNA and dsDNA are fractionated in an NaPO₄ buffer having a concentration of 0.15-0.17 M.

13. The method according to any of claims 1 to 12, wherein the adsorption chromatography in step e) is carried out in spin columns.

14. The gene structure, comprising a sequence according to SEQ ID No. 9 with at least one multiple cloning site comprising at least one rarely occurring recognition site for the restriction enzyme I-Ppol, and a primer-binder site and a T7-polymerase recognition site whose activity is regulated via the lac operator.

15. A vector, comprising at least one gene structure according to claim 14.

16. The use of the rarely occurring recognition site for the I-Ppol restriction endonuclease for preparing a gene structure according to claim 14 or a vector according to claim 15.

17. The use of the normalized gene bank prepared by a method according to any of claims 1 to 13 for the selection of genes coding for biocatalysts of soil-dwelling microorganisms.

## Revendications

1. Procédé d'établissement d'une banque de gènes normalisée constituée d'extraits d'acides nucléiques d'échantillons de sol, dans lequel :
a) on extrait des acides nucléiques d'êtres vivants contenus dans les échantillons de sol,
b) on réalise la fragmentation des acides nucléiques,
c) on réalise une quantification des fragments d'acides nucléiques par des colorants fluorescents,
d) on réalise une normalisation des fragments d'acides nucléiques en dénaturant d'abord les fragments d'acides nucléiques et en suivant le déroulement de la renaturation au moyen de colorants fluorescents,
e) lorsque la renaturation est terminée, on réalise par chromatographie par adsorption une séparation des acides nucléiques à double brin des acides nucléiques à simple brin, les espèces d'acides nucléiques contenues dans la fraction d'acides nucléiques à simple brin étant présentes à une fréquence sensiblement égale (normalisée) et
f) pour établir la banque de gènes, on clone dans un vecteur les espèces d'acides nucléiques normalisées.

2. Procédé selon la revendication 1, **caractérisé en ce que** l'on y réalise une isolation sélective des acides nucléiques des actinomycètes.

3. Procédé selon l'une des revendications 1 à 2, **caractérisé en ce que** lors de la fragmentation des acides nucléiques, on utilise de l'albumine non acétylée de sérum de bovin à des concentrations comprises entre environ 1 à 15 µg/µl, de préférence d'environ 2 à 12 µg/µl et de manière particulièrement préférable d'environ 10 µg/µl de milieu de restriction.

4. Procédé selon l'une des revendications 1 à 3, **caractérisé en ce que** les acides nucléiques fragmentés sont reliés par des ligands qui présentent au moins un emplacement de reconnaissance d'une endonucléase de restriction rarement présente.

5. Procédé selon l'une des revendications 1 à 4, **caractérisé en ce que** l'on utilise des ligands qui présentent un emplacement de reconnaissance de l'enzyme de restriction I-PpoI.

6. Procédé selon l'une des revendications 1 à 5, **caractérisé en ce qu'**à l'étape f), on utilise un vecteur qui présente au moins un emplacement de reconnaissance d'une endonucléase de restriction rarement présente compatible avec l'emplacement de reconnaissance situé sur les ligands.

7. Procédé selon l'une des revendications 1 à 6, **caractérisé en ce qu'**à l'étape c), la quantification des acides nucléiques extraits d'échantillons du sol et/ou leurs fragments s'effectue par des colorants fluorescents et de préférence par le SYBR-Green-I.

8. Procédé selon l'une des revendications 1 à 7, **caractérisé en ce qu'**à l'étape d), on suit l'évolution dans le temps de la renaturation des fragments d'acides nucléiques précédemment dénaturés par fluorimétrie à l'aide de colorants fluorescents spécifiques à l'ADN et de préférence avec le SYBR-Green-I.

9. Procédé selon l'une des revendications 1 à 8, **caractérisé en ce qu'**à l'étape e), la chromatographie par adsorption s'effectue au moyen d'hydroxyapatite.

10. Procédé selon l'une des revendications 1 à 9, **caractérisé en ce qu'**à l'étape e), la chromatographie par adsorption s'effectue par lots discontinus.

11. Procédé selon l'une des revendications 1 à 10, **caractérisé en ce que** l'on réalise la séparation de l'ADN à simple brin et de l'ADN à double brin entre 20 et 60° C, de préférence entre 20 et 30° C et de façon particulièrement préférable à 22° C.

12. Procédé selon l'une des revendications 1 à 11, **caractérisé en ce que** la séparation de l'ADN à simple brin et de l'ADN à double brin s'effectue dans un tampon de NaPO₄ à une concentration de 0,15 à 0,17 M.

13. Procédé selon l'une des revendications 1 à 12, **caractérisé en ce qu'**à l'étape e), la chromatographie par adsorption s'effectue dans des colonnes Spin.

14. Structure génique qui contient une séquence selon SEQ ID No:9 qui présente au moins un emplacement multiple de clonage qui contient au moins un emplacement de reconnaissance rarement présent de l'enzyme de restriction I-PpoI ainsi qu'un emplacement de liaison d'amorce et qu'un emplacement de reconnaissance d'une T7-polymérase dont l'activité est régulée par l'intermédiaire de l'opérateur Lac.

15. Vecteur qui contient au moins une structure génique selon la revendication 14.

16. Utilisation de l'emplacement de reconnaissance rarement présent de l'endonucléase de restriction I-PpoI pour préparer une structure génique selon la revendication 14 ou un vecteur selon la revendication 15.

17. Utilisation de la banque de gènes normalisée établie à l'aide d'un procédé selon l'une des revendications 1 à 13 pour la sélection de gènes qui codent pour des biocatalyseurs de micro-organismes vivant dans le sol.


### SEQUENCE LISTING

<110> BASF Aktiengesellschaft
<120> Verfahren zur Herstellung einer normalisierten Genbank aus Nukleinsäure-Extrakten von Bodenproben und deren Verwendung
<130> 1
<140> n
   <141> 2001-09-12
<160> 7
<170> PatentIn Ver. 2.1
<210> 1
   <211> 27
   <212> DNA
   <213> Artificial Sequence
<220>
   <223> Description of Artificial Sequence:Stuffer 5'-3' Richtung
<400> 1
   agctttaatg cggccgctgt gaatgcg 27
<210> 2
   <211> 21
   <212> DNA
   <213> Artificial Sequence
<220>
   <223> Description of Artificial Sequence:Stuffer 3'-5'-Richtung
<400> 2
   aattacgccg gcgacactta c 21
<210> 3
   <211> 29
   <212> DNA
   <213> Artificial Sequence
<220>
   <223> Description of Artificial Sequence:multiple Klonierstelle 5'-3'-Richtung
<400> 3
   gatcccgggc atgctctctt aaggtagcg 29
<210> 4
   <211> 29
   <212> DNA
   <213> Artificial Sequence
<220>
   <223> Description of Artificial Sequence:multiple Klonierstelle 3'-5'-Richtung
<400> 4
   ggcccgtacg agagaattcc atcgcctag 29
<210> 5
   <211> 50
   <212> DNA
   <213> Artificial Sequence
<220>
   <223> Description of Artificial Sequence:T7-Promotor 5'-3'-Richtung
<400> 5
   ggccgctaat acgactcact ataggggaat tgtgagcgga taacaattcc g 51
<210> 6
   <211> 51
   <212> DNA
   <213> Artificial Sequence
<220>
   <223> Description of Artificial Sequence:T7-Promotor 3'-5'-Richtung
<400> 6
   cgattatgct gagtgatatc cccttaacac tcgcctattg ttaaggccta g 51
<210> 7
   <211> 165
   <212> DNA
   <213> Artificial Sequence
<220>
   <223> Description of Artificial Sequence:Genstruktur pSCR
<400> 7
   ataagcttta atgcggccgc taatacgact cactataggg gaattgtgag cggataacaa 60
   ttccggatcc cgggcatgct ctcttaaggt agcggatccg gaattgttat ccgctcacaa 120
   ttcccctata gtgagtcgta ttagcggccg ctgtgaatgc gcaaa                 165

### SEQUENCE LISTING

<110> BASF Aktiengesellschaft
<120> Verfahren zur Herstellung einer normalisierten Genbank aus Nukleinsäure-Extrakten von Bodenproben und deren Verwendung
<130> PCT/EP 02/10510
<160> 9
<170> PatentIn version 3.1
<210> 1
   <211> 27
   <212> DNA
   <213> artificial sequence
<220>
   <223> linker, 5'-3'
<400> 1
   ggtcatgaac tctcttaagg tagcatg 27
<210> 2
   <211> 24
   <212> DNA
   <213> artificial sequence
<220>
   <223> linker, 3'-5'
<400> 2
   tccagtactt gagagaattc catc 24
<210> 3
   <211> 27
   <212> DNA
   <213> artificial sequence
<220>
   <223> stuffer, 5'-3'
<400> 3
   agctttaatg cggccgctgt gaatgcg 27
<210> 4
   <211> 21
   <212> DNA
   <213> artificial sequence
<220>
   <223> stuffer, 3'-5'
<400> 4
   aattacgccg gcgacactta c 21
<210> 5
   <211> 29
   <212> DNA
   <213> artificial sequence
<220>
   <223> multiple Klonierstelle 5'-3'
<400> 5
   gatcccgggc atgctctctt aaggtagcg 29
<210> 6
   <211> 29
   <212> DNA
   <213> artificial sequence
<220>
   <223> multiple Klonierstelle 3'-5'
<400> 6
   ggcccgtacg agagaattcc atcgcctag 29
<210> 7
   <211> 51
   <212> DNA
   <213> artificial sequence
<220>
   <223> T7-Promotor 5'-3'
<400> 7
   ggccgctaat acgactcact ataggggaat tgtgagcgga taacaattcc g 51
<210> 8
   <211> 51
   <212> DNA
   <213> artificial sequence
<220>
   <223> T7-Promotor 3'-5'
<400> 8
   cgattatgct gagtgatatc cccttaacac tcgcctattg ttaaggccta g 51
<210> 9
   <211> 165
   <212> DNA
   <213> artificial sequence
<220>
   <223> Genstruktur pSCR
<400> 9
   ataagcttta atgcggccgc taatacgact cactataggg gaattgtgag cggataacaa     60
   ttccggatcc cgggcatgct ctcttaaggt agcggatccg gaattgttat ccgctcacaa    120
   ttcccctata gtgagtcgta ttagcggccg ctgtgaatgc gcaaa                    165